(19) Europäisches Patentamt
European Patent Office
Office européen des brevets

(11) Publication number: **0 510 265 A1**

# (12) EUROPEAN PATENT APPLICATION

(21) Application number: **91303632.3**

(22) Date of filing: **23.04.91**

(51) Int. Cl.5: **C08G 59/10**, C08G 59/18, C08G 59/32, C08L 63/00

(43) Date of publication of application:
**28.10.92 Bulletin 92/44**

(84) Designated Contracting States:
**DE FR GB**

(71) Applicant: **TEXACO CHEMICAL COMPANY**
**3040 Post Oak Boulevard**
**Houston, Texas 77056(US)**

(72) Inventor: **Lin, Jiang-Jen**
**15031 Rock Knoll**
**Houston, Texas 77083(US)**
Inventor: **Speranza, George Phillip**
**2800 Silverleaf**
**Austin, Texas 78757(US)**

(74) Representative: **Brock, Peter William et al**
**UROUHART-DYKES & LORD 91 Wimpole Street**
**London W1M 8AH(GB)**

## (54) Liquid polyglycidyl ethers and amine terminated derivatives thereof.

(57) Liquid compounds suitable for preparing epoxy resins have the formula (I) and (VII)

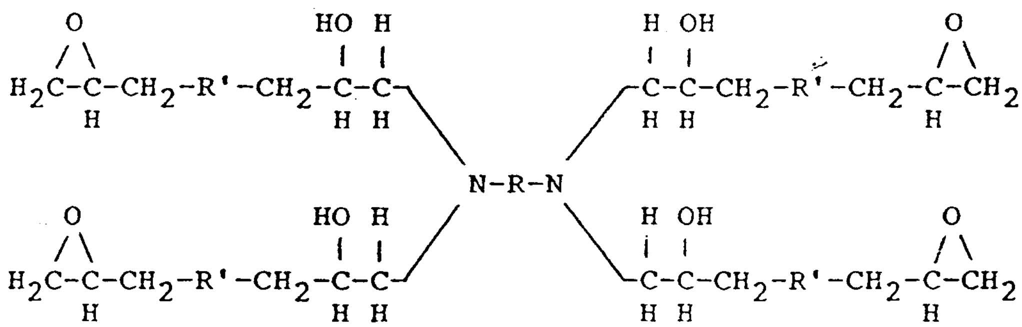

(I)

(VII) 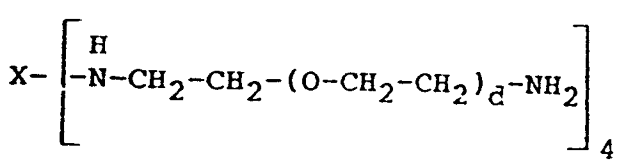

wherein d is 1 to 4 and
wherein X represents a group having the formula:

EP 0 510 265 A1

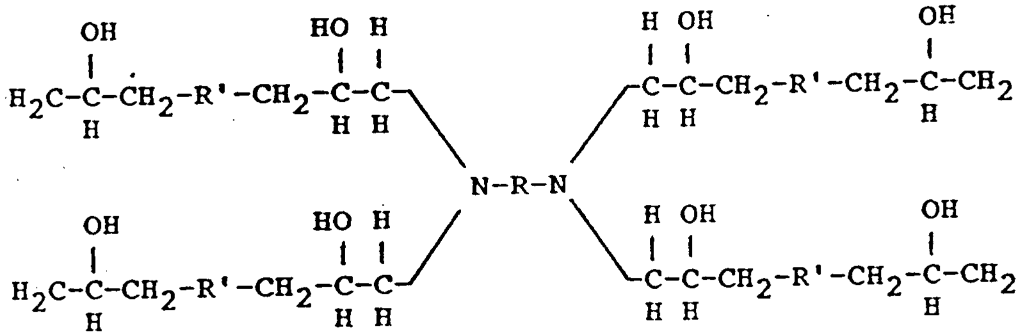

(VIII)

R is the residue of a fatty acid dimer diamine containing about 36 carbon atoms, or a polyoxyalkylene group having the formula (II) or (V)

(II)

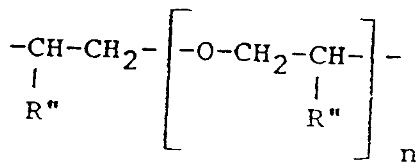

(V)

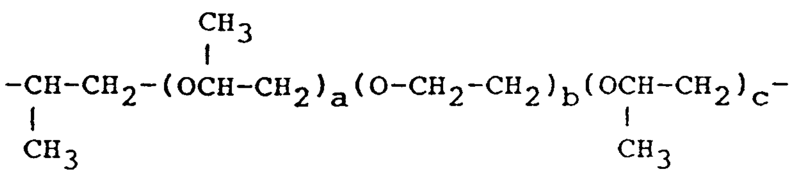

in which R'' hydrogen, methyl or ethyl,
n is 2 to 35, a+c is 2 to 10, and b is 1 to 50; and
R' represents a glycidyl ether residue having the formula:

(III)

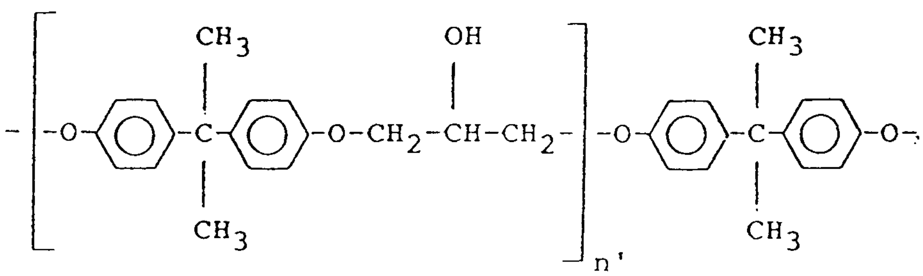

wherein n' is 0 or 1 to 2

This invention relates to liquid glycidyl ethers and amine terminated derivatives thereof. More particularly, this invention relates to liquid compounds prepared from diglycidyl ethers of Bisphenol A and polyoxyalkylene diamines, as hereinafter defined. In another embodiment, this invention relates to liquid amine terminated derivatives prepared by reacting polyoxyethylene diamines with the above-mentioned epoxy terminated derivatives of glycidyl ethers of Bisphenol A and polyoxyalkylene diamines.

This invention also relates to methods for preparing the above-mentioned compounds.

Both the epoxy-terminated derivatives and the amine-terminated derivatives of the present invention are useful in the preparation of epoxy resins.

It is well known that various polyamine compounds are widely used as raw materials for hardening agents of epoxy resins. For these resins, typical examples of useful polyamine compounds are aliphatic amines such as ethylenediamine, diethylenetriamine, triethylenetetramine, tetraethylenepentamine, pentaethylenehexamine and hexamethylenediamine; aromatic amines such as phenylenediamine, diaminophenylmethane and diaminophenylsulfone; aliphatic amines having an aromatic ring, such as xylylenediamine; and alicyclic amines such as bisaminomethylcyclohexane or isophoronediamine. These polyamine compounds each have reactive hydrogens in their amino groups and they are used for various purposes. Further, polyamine compounds which have been modified in various ways, suitable for the specific polyamine compound, have found a variety of uses and the modified compounds are used as hardening agents for epoxy resins.

Adducts from amines and diepoxides have long been used in industry as curing agents for epoxy resins.

It is known from US-A-3538184 that a polyglycidyl ether can be reacted with hexamethylenediamine to form solvent-free liquid adducts of polyepoxides and polyamines which are suitable for use as curing agents for epoxy resins.

US-A-3629181 describes a curing agent which comprises the adduct from a polyglycidyl ether of a polyphenyl and a cycloaliphatic or cycloaliphatic-aliphatic di-primary diamine, in which at least one of the primary amino groups is bonded to an endocyclic carbon atom of a cycloaliphatic ring.

US-A-3996175 describes moulding materials from epoxide resins based on Bisphenol A or on other bis- or polyphenols and epichlorohydrin which contain aromatic amines as curing agents.

US-A-4348505 discloses the preparation of amine adduct curing agents utilizing epoxides having a functionality greater than two which dramatically enhance the chemical resistance properties of the cured epoxy products over results obtained from either free amines or the conventional amine adducts.

US-A-4540750 discloses that the preparation of diethyl toluene diamine adduct curing agents with epoxides having a functionality of at least two, enhances the performance characteristics of cured epoxy and urethane products.

US-A-4578412 relates to modified (i.e., extended) amine curing agents which are prepared by dissolving a solid elastomeric polyurethane, such as RIM polyurethane, in an amine of the type used for curing epoxy resins.

A novel polyamine compound made by reacting m-xylyene diamine and epichlorohydrin in the presence of an alkali, and which is useful as a hardening agent for epoxy resins, is disclosed in US-A-4605765.

US-A-4608404 describes compositions which contain specific groups of oligomeric diamine hardeners and epoxy compounds which, when combined with structural fibres, produce composites which have improved tensile properties, high compressive strengths and improved impact resistance and, in addition, demonstrate low moisture adsorption.

In an article titled "High Solids Epoxy/Polyamide Coatings", V. Brytus discusses a new polyamide hardener which overcomes the compatibility problem brought about by reducing the molecular weight of epoxy/polyamidoamine maintenance coatings in an attempt to lower the volatile organic content, (Journal of Coatings Technology, Vol. 58, No. 740, September 1986). The polyamide has controlled levels of imidazoline and other constituents having an affinity for the Bisphenol A moiety. A two-step method of producing N,N'-dimethyl diamine compounds for use as epoxide polyaddition compounds is described in US-A-4623746. The resins formed using this compound as a curing agent can be produced with improved strength, toughness and hot/wet properties.

US-A-4636535 discloses curable compositions comprising epoxide prepolymers and polyamidobenzoates, alone or combined with reinforcements such as graphite fibres.

US-A-4680341 discloses epoxy resin systems which exhibit good tensile properties and good moisture sensitivity, and which are made by copolymerizing tetraglycidates with a polyamine curing agent.

From the preceding discussion it is apparent that compositions with a variety of properties which are the product of a wide range of amine and epoxide components are useful in the field of epoxy curing resins.

This invention provides compounds having the formula:

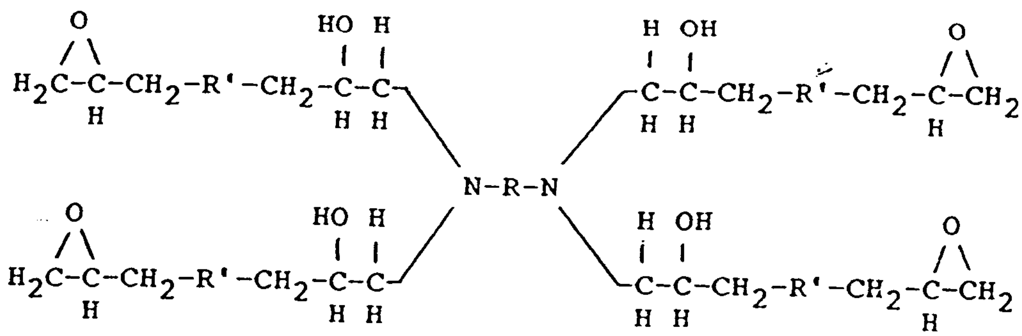

(I)

wherein:
R is the residue of a fatty acid dimer diamine containing about 36 carbon atoms, or a polyoxyalkylene group having the formula (II) or (V)

(II)

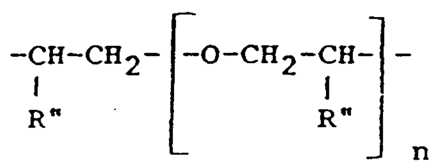

or

(V)

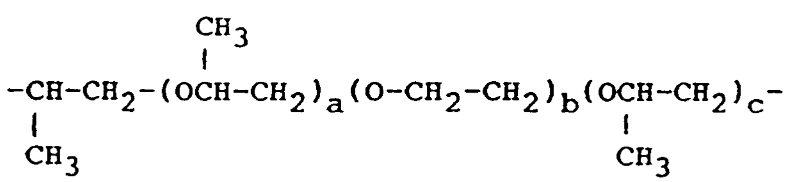

in which R" hydrogen, methyl or ethyl,
n is 2 to 35, a+c is 2 to 10, and b is 1 to 50; and
R' represents a glycidyl ether residue having the formula:

(III)

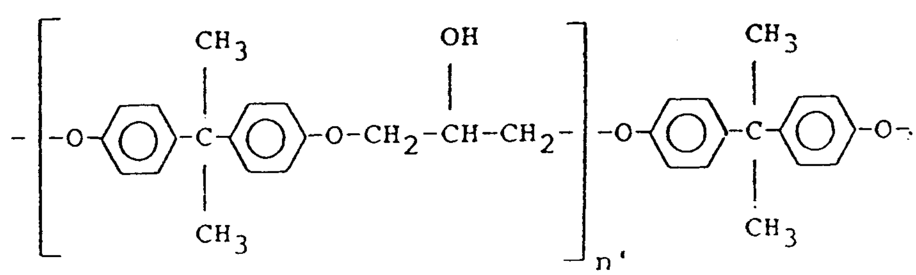

wherein n' is 0 or 1 to 2.

Another embodiment of this invention provides an amine terminated compound having the formula:

(VII)

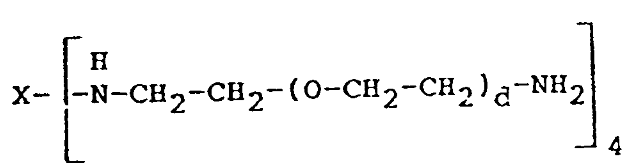

wherein d is 1 to 4 and
wherein X represents a group having the formula

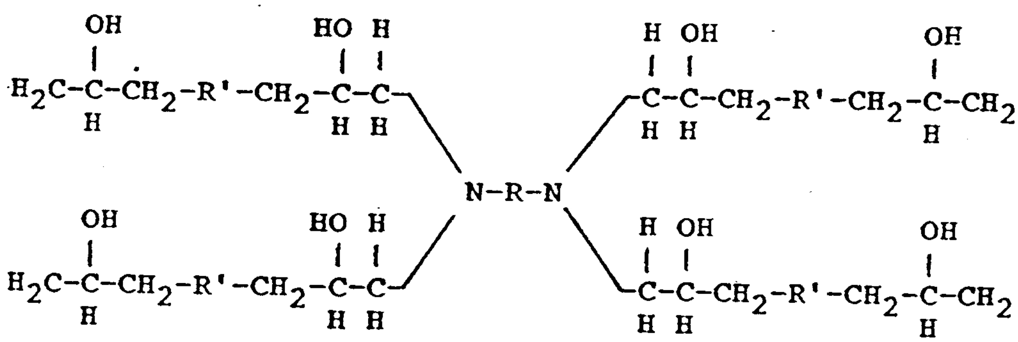

(VIII)

wherein R and R' have the meanings given above. This invention also provides a method of making a compound having formula (I) which comprises:

a) mixing a solution of a diglycidyl ether having the formula:

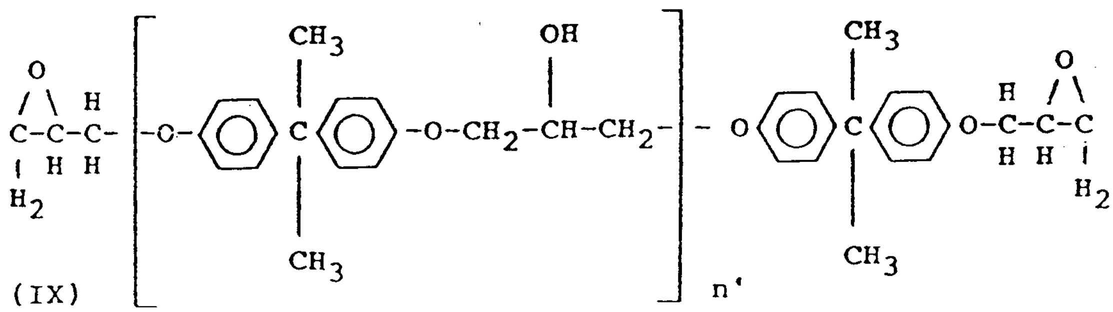

(IX)

wherein n' has the meaning given above, in acetone or methyl ethyl ketone as solvent with a polyoxyalkylene diamine of the formula

$H_2N\text{-}R\text{-}NH_2$

wherein R has the meaning given above, in a molar ratio of from 4 to 5 moles of diglycidyl ether per mole of polyoxyalkylene diamine, and

b) heating the resulting mixture with agitation at a temperature of 80 to 160 ° C for 1 to 4 hours, to react said diglycidyl ether quantitatively with said polyoxyalkylene diamine, and to volatilize the solvent.

According to another embodiment, after reaction of the glycidyl ether and the diamine, and volatilization of the solvent, a polyoxyethylene diamine having the formula:

(XII) $$H_2N\text{-}CH_2\text{-}CH_2\text{-}\left[\text{-}O\text{-}CH_2\text{-}CH_2\text{-}\right]_d\text{-}NH_2$$

wherein d has the meaning given in above, is added in a mole ratio from 8.5 to 10 mols of polyoxyethylene diamine per mole of diamine initially charged, followed by heating at a temperature of 100 to 150 ° C for 0.5 to 5 hours, to form an amine terminated derivative having the formula (VII).

Both the epoxy terminated derivatives and the amine terminated derivatives of the present invention, although with comparatively high molecular weights in the order of from 2000 to 10,000, are liquids at ambient temperatures. This is surprising, because the normally expected products from such reactants would be gels. Polyether amines are standard reagents for the curing of epoxy resins to solid products.

It has been discovered in accordance with the present invention that liquid epoxy terminated derivatives

and liquid amine terminated derivatives having the formulas set forth above can be prepared by following the method of the present invention.

An advantage of both the epoxy-terminated and the amine-terminated compounds of the invention is that the products are liquid at ambient temperatures and can be used as epoxy curing agents in preparing epoxy resins wherein added flexibility is an important property.

If the symbol "E" represents a diglycidyl ether of Bisphenol A, the symbol "A" represents the polyoxyalkylene diamine or fatty acid dimer diamine and the symbol "$A^+$" represents the polyoxyethylenediamine, then the reactions and reaction products of the present invention can be visualized thusly:

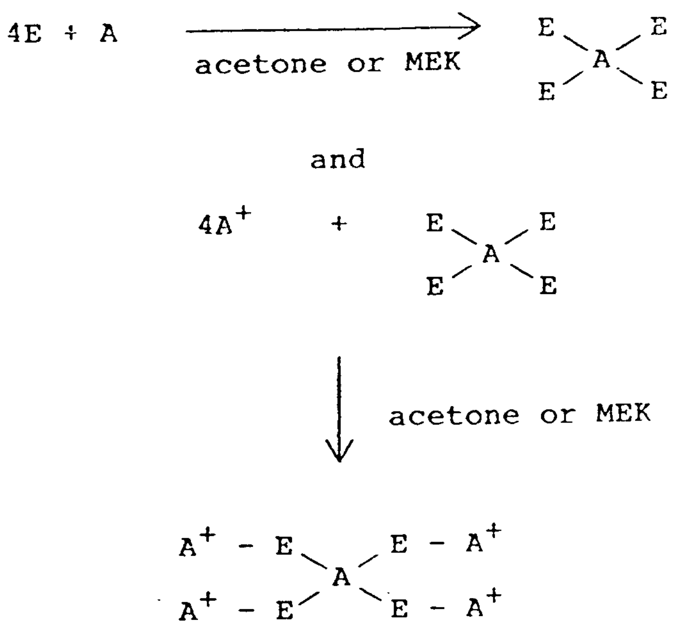


As can be more easily seen from the foregoing, the process and products of the present invention provide an improvement in the preparation of epoxy resins wherein a significant portion of the Bisphenol A component is "prereacted" before the final epoxy resin formation step to provide liquid intermediates that are easy to prepare, easy to store and easy to use.

Key factors that are involved include the discovery that it is necessary to use either acetone or methyl ether ketone as a solvent in the process of the present invention and the discovery of the need to use a polyoxyethylene diamine in the second step of the process.

The diglycidyl ethers of Bisphenol A to be used in accordance with the present invention are diglycidyl ethers having the formula:

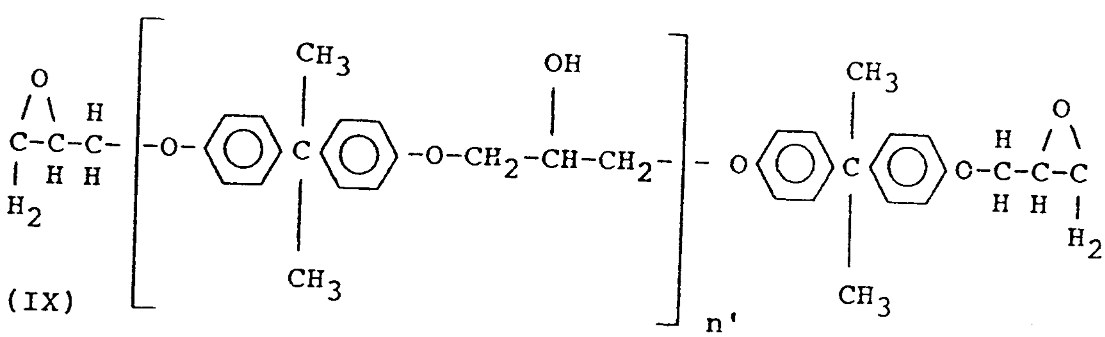

(IX)

wherein n' represents 0 or 1 to 2.

The polyoxyalkylene diamines to be used as starting material in accordance with the present invention are polyoxyalkylene diamines having the formula:

(X) 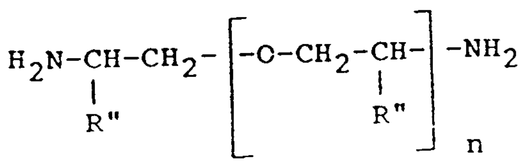

wherein n is 2 to 35 and wherein R" represents hydrogen, methyl or ethyl.

Representative products having this structural formula include polyoxypropylene diamines (wherein R" is methyl) having an average molecular weight of about 230 wherein n is between 2 and 3 (Jeffamine$^R$ D-230 amine), polyoxypropylene diamines having an average molecular weight of about 2,000 wherein n is between 5 and 6 (Jeffamine$^R$ D-400 amine), and a polyoxypropylene diamine product having an average molecular weight of about 2,000 wherein n is about 33 (Jeffamine$^R$ D-2000 amine).

Another appropriate class of polyoxyalkylene diamines, containing both ethylene oxide and propylene oxide, which may be used are polyoxypropylene diamines that are sold by the Texaco Chemical Company as Jeffamine$^R$ ED-series products having the formula:

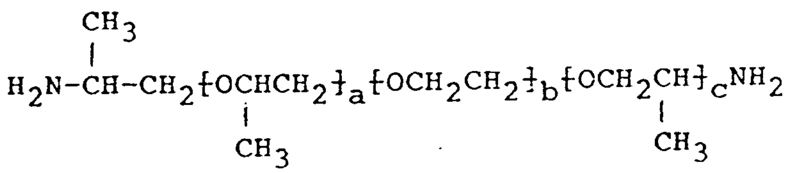

wherein a + c is 2 to 10 and b is 1 to 50.

In another embodiment, fatty acid dimer diamines containing about 36 carbon atoms can be used instead of the polyoxyalkylene diamines.

Examples of products having this general formula include a commercial product having an average molecular weight of about 600, where b is about 8.5 and a+c is about 2.5 (Jeffamine $^R$ ED-600), a commercial product having an average molecular weight of about 900 wherein a + c is again about 2.5, but the value of b is about 15.5 (Jeffamine$^R$ ED-900). Other examples are those wherein a + c is about 2.5, including a product having an average molecular weight of about 2,000 wherein b is about 40 (Jeffamine $^R$ ED-2001).

The polyoxyethylene diamine starting material to be used in accordance with the present invention has the formula:

(XII) $H_2N\text{-}CH_2\text{-}CH_2\text{-}[\text{-}O\text{-}CH_2\text{-}CH_2\text{-}]_d\text{-}NH_2$

wherein d is 1 to 4.

Representative examples of such polyoxyethylene diamines include:

Jeffamine$^R$ EDR-148, which is an amine terminated triethylene glycol having the formula:

$H_2N\text{-}CH_2CH_2\text{-}O\text{-}CH_2CH_2\text{-}O\text{-}CH_2CH_2\text{-}NH_2$

and Jeffamine$^R$ EDR-192, which is an amine terminated tetraethylene glycol having the formula:

$H_2N\text{-}CH_2CH_2\text{-}O\text{-}CH_2CH_2\text{-}O\text{-}CH_2CH_2\text{-}O\text{-}CH_2CH_2\text{-}NH_2$

In accordance with the present invention, epoxy terminated derivatives of diglycidyl ethers of Bisphenol A and polyoxyalkylene diamines are prepared by the following sequence:

A diglycidyl ether of Bisphenol A having formula (IX) is dissolved in either acetone or methylethyl ketone. Acetone is the preferred solvent and given the best results.

The thus prepared solution is added to a reaction vessel and a polyoxyalkylene diamine having formula (X) is added, the amount of polyoxyalkylene diamine added being such that there are 4 to 5 moles of the diglycidyl ether per mole of polyoxyalkylene diamine. The preferred ratio is about 4.5 moles of the diglycidyl ether per mole of polyoxyalkylene diamine.

The thus formed reaction mixture is heated with agitation at a temperature of 80 to 160 °C for 1 to 4 hours, and sufficient to permit the diglycidyl ether to react quantitatively with the polyoxyalkylene diamine

and substantially completely to volatilize the ketone solvent. Thereafter, if desired, the epoxy terminated derivative can be recovered from the reaction mixture and will have the formula (I) given above.

In accordance with another embodiment of the present invention, an epoxy terminated derivative of Bisphenol A, prepared in the manner just described, is further reacted with a polyoxyethylene diamine having the formula (XII) given above.

This can be accomplished by further adding to the reaction mixture described above 4 moles or more, such as 4 to 10 moles, more preferably 8.5 to 10 moles, of the polyoxyalkylene per mole of said polyoxyalkylene diamine starting material used in the preparation of the epoxy terminated diglycidyl ether. In this situation, the epoxy terminated diglycidyl derivative is, in effect, an intermediate reaction product and, when mixed with the polyoxyethylene diamine, provides a second reaction mixture.

The second reaction mixture is heated at a temperature of 100 to 150°C for 0.5 to 5 hours, and thereafter a tetrafunctional amine derivative having the formula (VII) is recovered.

It is known to use amines such as aliphatic or aromatic amines for curing 1,2-epoxy resins as shown, for example, by US-A-4139524 and -4162358, and in the textbook "Handbook of Epoxy Resins" by H. Lee and K. Neville, McGraw-Hill Book Company, 1967.

Generally the vicinal epoxide compositions that can be cured using the curing agents of this invention are organic material having an average of more than one reactive 1,2-epoxide group. These polyepoxide materials can be monomeric or polymeric, saturated or unsaturated, aliphatic, cycloaliphatic, aromatic or heterocyclic, and may be substituted if desired with other substituents besides the epoxy groups, e.g. hydroxyl groups, ether radicals, or halogenated phenyl groups.

The most widely used epoxy resins are diglycidyl ethers of bisphenol A:

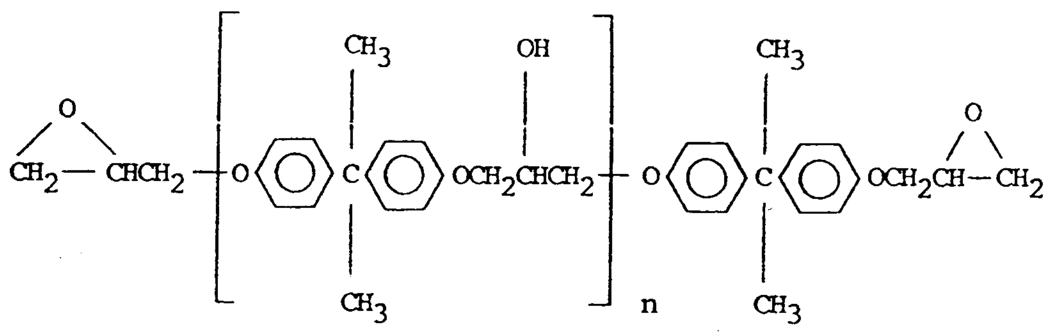

where n is 1 to 2.

These epoxides are only representative of the broader class of epoxide compounds that are useful in making epoxy resins. A widely used class of polyepoxides that can be cured according to the practice of the present invention includes the resinous epoxy polyethers obtained by reacting an epihalohydrin, such as epichlorohydrin, with either a polyhydric phenol or a polyhydric alcohol. An illustrative, but by no means exhaustive, listing of suitable dihydric phenols includes 4,4'-isopropylidene bisphenol, 2,4'-dihydroxydiphenylethylmethane, 3,3'-dihydroxydiphenyl-diethylmethane, 3,4'-dihydroxydiphenylmethylpropylmethane, 2,3'-dihydroxydiphenylethylphenylmethane, 4,4-dihydroxydiphenylmethane, 4,4-dihydroxydiphenylbutylphenylmethane, 2,2'-dihydroxydihenylditolylmethane, and 4,4'-dihydroxydiphenyltolylmethyl-methane. Other polyhydric phenols which may also be co-reacted with an epihalohydrin to provide these epoxy polyethers are such compounds as resorcinol, hydroquinone, and substituted hydroquinones, e.g. tertbutylhydroquinone.

Among the polyhydric alcohols that can be co-reacted with an epihalohydrin to provide the resinous epoxy polyethers are such compounds as ethylene glycol, propylene glycol, butylene glycols, pentane diols, bis (4-hydroxycyclohexyl)dimethylmethane, 1,4-dimethylolbenzene, glycerol, 1,2,6-hexanetriol, trimethylolpropane, mannitol, sorbitol, erythritol, pentaerythritol, their dimers, trimers and higher polymers, (e.g. polyethylene glycols or polypropylene glycols), triglycerol, dipentaerythritol, polyallyl alcohol, polyhydric thioethers (such as 2,2', 3,3'-tetrahydroxydipropylsulfide), mercapto alcohols (such as α-monothioglycerol or α,α'dithioglycerol), polyhydric alcohol partial esters (such as monosterin or pentaerythritol monoacetate) and halogenated polyhydric alcohols such as the monochlorohydrins of glycerol, sorbitol or pentaerythritol.

Another class of polymeric polyepoxides that can be cured by means of the above-described curing agents includes the epoxy novolac resins obtained by reacting, preferably, in the presence of a basic catalyst, e.g. sodium or potassium hydroxide, an epihalohydrin, such as epichlorohydrin, with the resinous

condensate of an aldehyde, e.g. formaldehyde and either a monohydric phenol, phenol itself, or a polyhydric phenol. Further details concerning the nature and preparation of these epoxy novolac resins can be obtained in Lee, H. and Neville, K. "Handbook of Epoxy Resins".

It will be appreciated by those skilled in the art that the polyepoxide compositions that can be cured according to the practice of the present invention are not limited to the above described polyepoxides, but these polyepoxides are to be considered merely as being representative of the class of polyepoxides as a whole.

The amount of curing agent that is employed in curing polyepoxide compositions will depend on the amine equivalent weight of the curing agent employed. The total number of equivalents of amine group is preferably from 0.8 to 1.2 times the number of epoxide equivalents present in the curable epoxy resin composition, with the stoichiometric amount being most preferred.

Various conventionally employed additives can be mixed with these polyepoxide-containing compositions before final cure. For example, in certain instances it may be desired to add minor amounts of other co-catalysts, or hardeners, along with the curing agent system herein described. Conventional pigments, dyes, fillers, flame retarding agents and other compatible natural and synthetic resins can also be added.

The invention will be further illustrated by the following specific Examples:

Example 1

Products from Jeffamine D-400 and Epon 828 (1:4.5 molar ratio).

To a 1 litre, three-necked flask equipped with a thermometer, Dean-Stark trap, stirrer and nitrogen line was charged Jeffamine D-400 (60g, 0.15M) and Epon 828 (253g, 0.675 mole) in acetone (100 ml). The mixture was heated at 100 °C for 2 hours to remove acetone and to obtain a transparent, light-yellow semi-solid.

Example 2

Products from Jeffamine D-400 and Epon 828 (1:3 molar ratio) (Comparative).

To a 500ml three-necked flask equipped with a thermometer, Dean-Stark trap, stirrer and $N_2$ line, was charged D-400 (20g, 0.05 mole) and Epon 828 (56g, 0.15 mole) in acetone (50ml). The mixture was heated at reflux temperature. During the process of removing acetone, the mixture turned into a gel. The result shows that ratios of D-400 below 4 form gelled products.

Example 3

Products from Jeffamine D-400 and Epon 828 (1:2 molar ratio) (Comparative)

To a 250 ml three-necked flask equipped with a thermometer, Dean-Stark trap, mechanical stirrer and nitrogen inlet line, was charged Jeffamine D-400 (30g, 0.075 mole) and Epon 828 (56g, 0.15 mole) in acetone (50ml). The mixture was heated at 67 to 75 °C for 2 hours. While reaction temperature was raising to 90 °C gel formation was observed. This indicated the 1:2 molar ratio of D-400 to Epon 828 was not suitable for avoiding gel formation.

Example 4

Products from Epon 828 and Jeffamine ED-2001 with Acetone (4:1 molar ratio)

To a 250ml three-necked flask equipped with a thermometer, stirrer, Dean-Stark trap and $N_2$ line, was charged Epon 828 (75g 0.2 moles), and ED-2001 (100g, 0.05 moles) in acetone (50ml). The mixture was heated to remove solvent to 140 °C for ca. 2 hours. The resulting material was an attractive, transparent, light-colored liquid.

Example 5 Products from Dimer Amine and Epon 828 in Acetone (1:6 molar ratio)

To a 250ml three-necked flask equipped with a thermometer, Dean-Stark trap, stirrer and nitrogen inlet line was charged a 36 carbon atom dimer amine having a molecular weight of about 590 (29.6g, 0.05M) and

Epon 828 (112g, 0.3M) in acetone (100ml). The mixture was heated slowly to remove acetone. The mixture was then heated at 150 °C under vacuum for ca. 1 hour. The resulting product was a viscous brown liquid (139g).

Example 6

Products from Epon 828 and Jeffamine ED-2001 (4:1 molar ratio) (Comparative)

To a 250ml three-necked flask equipped with a thermometer, mechanical stirrer and $N_2$ line was charged Epon 828 (Shell product, 75g, 0.2 moles) and Jeffamine ED-2001 (100g 0.05 moles). No acetone was used. The mixture was heated to 100-115 °C for about 2 hours. Gel formation was observed. The presence of acetone is the important factor for preparing a non-gelled product.

Example 7

Products from Dimer Amine and Epon 828 without using Acetone Solvent (Comparative)

To a 250ml three-necked flask equipped with a thermometer, Dean-Stark trap, stirrer and $N_2$ line was charged dimer amine (29.6g, 0.05M) and Epon 828 (112g, 10.3M). The mixture was heated at 100 °C for 2 hours. The product was a rubbery, gel-like material. The experiment demonstrated the importance of acetone.

Example 8

Products from Jeffamine D-2000 and Epon 828 (1:4.5 molar ratio)

to a litre three-necked flask equipped with a thermometer, Dean-Stark trap, stirrer and nitrogen line, was charged D-2000 (200g, 0.1M) Epon 828 (168g, 0.45) and acetone (100ml). The mixture was heated to reflux, the acetone was removed and then the product was heated at 120 °C for 2 hours. A transparent liquid product was obtained, whose amine content was 0.53 meq/g; total acetylatables 4.11 meq/g, and viscosity 14,000 cs/25 °C.

Example 9

Usage of Product

The product from Example 8 and EDR-148 (1.0g) were mixed carefully and poured into a mould. The liquid mixture was cured over-night at 80 °C to afford a flexible new material with high integrity.

Example 10

Products from D-2000-Epon 828-EDR-148 (1:4:9 molar ratio)

To a 500ml three-necked flask equipped with a thermometer, stirrer, Dean-Stark trap and $N_2$ line, was charged Jeffamine D-2000 (100g, 0.05M) and Epon 828 (74.8g, 0.2 mole) in acetone (50ml). The mixture was heated at 70-88 °C to remove acetone for one hour. Then, Jeffamine EDR-148 (66.6g, 0.45 mole) in acetone (30ml) was added. The final mixture was heated slowly to 120-130 °C and subjected to vacuum for ca. 1 hour. The resulting product (237g) was a yellowish-brown liquid having analyses of total amine 4.01 meq/g, hydroxyl number 5.32 meq/g and viscosity 5,500 cs/50 °C.

Example 11

Products from D-400-Epon 828-EDR-148 (1:4.5:10 molar ratio)

To a 500ml three-necked flask equipped with a thermometer, stirrer, Dean-Stark trap and $N_2$ line, was charged Jeffamine D-400 (20g, 0.05 mole) in acetone (20ml), and Epon 828 (84g, 0.225 mole) in acetone (80ml). The mixture was heated slowly to remove acetone over a two hour period. Then, Jeffamine EDR-148 (74g, 0.5 mole) in acetone (70ml) was added. The process of removing solvent was repeated. The mixture

was heated at 130°C under reduced pressure. The resulting product (185g) was viscous, transparent brown liquid having a viscosity 23,000 cs/50°C, a total amine content 5.82 meq/g and a hydroxyl number 7.48 meq/g.

Example 12

Products from D-400-Epon 828-EDR-148 (1:2:2 molar ratio) (Comparative)

To a 500ml three-necked flask equipped with a thermometer, Dean-Stark trap, mechanical stirrer and nitrogen inlet line, was charged Jeffamine D-400 (60g, 0.15 mole) in acetone (30g) and Epon 828 (112g, 0.30 moles) in acetone (100g). The mixture was heated at 70-80°C for one hour. Then Jeffamine EDR-148 (44.4g 0.30 moles) in acetone (40g) was added and heating was continued at 70-80°C. A gel material was obtained after acetone was removed. This experiment demonstrated the importance of the molar ratio of D-400-Epon 828-EDR-148.

Example 13

Products from D-200-Epon 828-EDR-192 (1:3:4 molar ratio)

To a 500ml three-necked flask equipped with a thermometer, stirrer, Dean-Stark trap and $N_2$ inlet line, was charged Jeffamine D-2000 (160g, 0.08 mole) in acetone (60g) and Epon 828 (90g, 0.24 mole) in acetone (90g). The mixture was heated to reflux and then to 100°C. Acetone was removed during the process. Then, EDR-192 (74g 0.385 mole ) in acetone (50ml) and water (50ml) was added. The process of removing solvent was repeated by heating the mixture to 130°C for ca. 2 hours. The resulting product was viscous liquid, having a viscosity of 22,000 cs/50°C, a total amine content of 2.79 meq/g and a hydroxyl number of 5.18 meq/g. The molar ratio of amine to epoxide is the important factor for controlling the gelling characteristics of the product.

Example 14

Products from D-2000-Epon 828-EDR-192 (1:4.5:5.0 molar ratio)

To a 500ml three-necked flask equipped with a thermometer, stirrer, Dean-Stark trap and $N_2$ line, was charged D-2000 (100g, 0.05 mole) and Epon 828 (84g, 0.225 mole) in acetone (80 ml). The mixture was heated to reflux temperature and acetone was removed. Then, EDR-192 (48g, 0.25 mole) in acetone (50ml) was added During the process of removing acetone, gel formation was observed. This experiment defined the limitations of molar ratio of Jeffamine amine(I)-Epon 828-Jeffamine amine(II).

Example 15

Products from D-2000-Epon 828-EDR-192 (1:2:2 molar ratio) (Comparative)

To a resin flask equipped with a thermometer, Dean-Stark trap, stirrer and $N_2$ inlet line, was charged D-2000 (160g, 0.08 mole) in acetone (60) and Epon 828 (60g, 0.16 moles) in acetone (60g). The mixture was heated at 70-100°C for four hours while removing acetone through a Dean-Stark trap. Then EDR-192 (31g. 0.16 moles) in acetone (30g) and water (15g) was added. The mixture was heated slowly to about 100°C and then at 130°C for four hours under aspirator vacuum. The recovered product was a rubbery brown solid.

Example 16

Products from D-2000-Epon 828-EDR-148 (1:2:2 molar ratio) (Comparative)

To a 500ml three-necked flask equipped with a thermometer, Dean-Stark trap, mechanical stirrer and nitrogen inlet line, was charged Jeffamine D-2000 (160g, 0.08 mole), Epon 828 (60g, 0.16 mole) and acetone (60g). The mixture was heated at 50-60°C for 2 hours. A sample of 19g was taken for analysis. Then Jeffamine EDR-148 (22g, 0.149 mole) in acetone (20g) was added. The reaction temperature was raised to remove acetone until dryness. The resulting material was a milky-white solid. The total amine

analysis showed 1.85 meq/g. After standing at room temperature, the product became two layers of liquid (brown, about 5 wt.% top layer) and solid (soft, milky-white, about 95 wt.%, bottom layer (indicating that the mixture was not homogeneous.

Example 17

Products from D-2000-Epon 828-EDR-148 (1:4.5:6.75 molar ratio)

To a 1 litre, three-necked flask equipped with a thermometer, stirrer, Dean-Stark trap and nitrogen line, was charged product from Example 16 (184g) and EDR-148 (50g, 0.338 mole) in acetone (50ml). The mixture was heated to remove acetone. During the process, gel formation took place.

Example 18

Usage of Product

The product of Example 10 (21.6g) and Epon 828 (20g) were mixed well and poured into a mould and cured at 60°C for 2 hours to give a flexible, tough material.

It is noted that:

1) In order to avoid gel product, the proper ratio of amine to epoxy resin is required. The first step requires at least a 1:4 molar ratio of amine to epoxide.

2) In the second step, reaction of tetrafunctional epoxy resin with active Jeffamine amine, a large excess of EDR-148 or EDR-192 was needed.

3) Acetone or a low boiling ketone is essential for preparing these products.

## Claims

**1.** A compound having the formula:

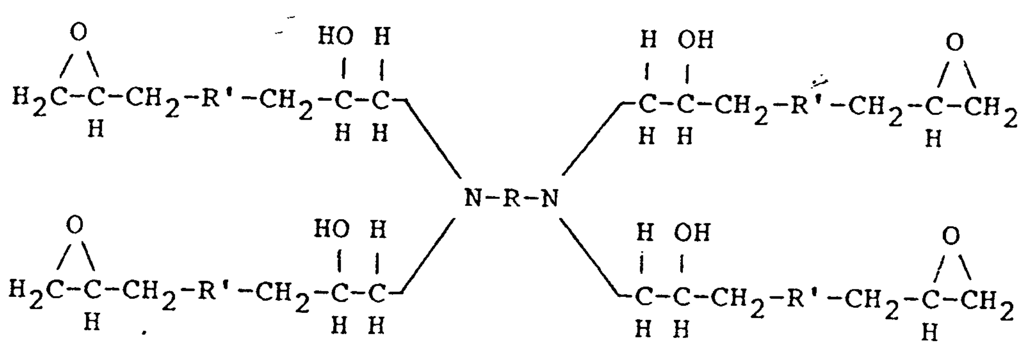

(I)

wherein:

R is the residue of a fatty acid dimer diamine containing about 36 carbon atoms, or a polyoxyalkylene group having the formula (II) or (V)

(II)

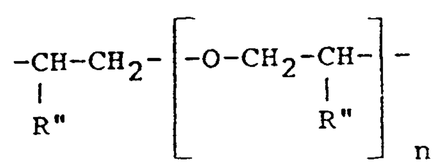

or

(V) 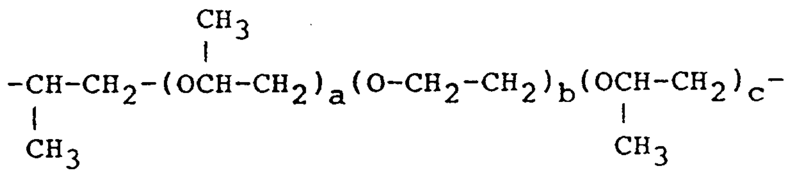

in which R'' hydrogen, methyl or ethyl,
n is 2 to 35, a + c is 2 to 10, and b is 1 to 50; and
R' represents a glycidyl ether residue having the formula:

(III) 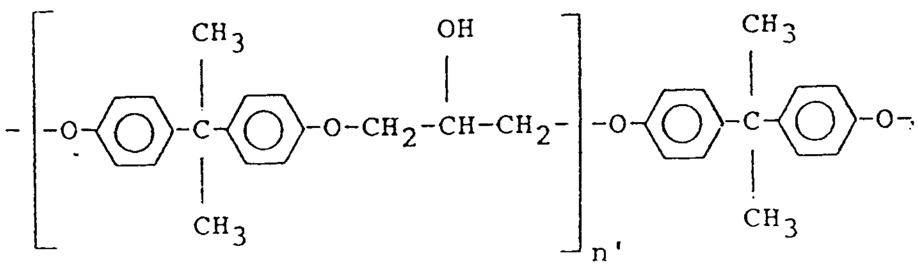

wherein n' is 0 or 1 to 2

2. A compound according to Claim 1 characterized in that R represents an oxypropylene group having the formula:

(IV) 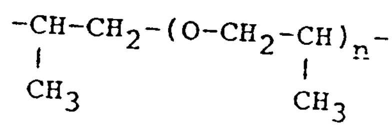

wherein n is 2 to 35.

3. An amine terminated compound having the formula:

(VII) 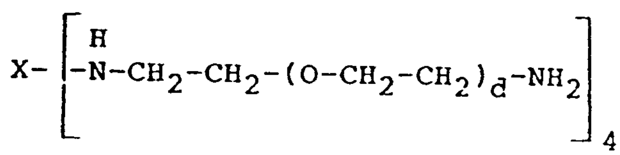

wherein d is 1 to 4 and
wherein X represents a group having the formula:

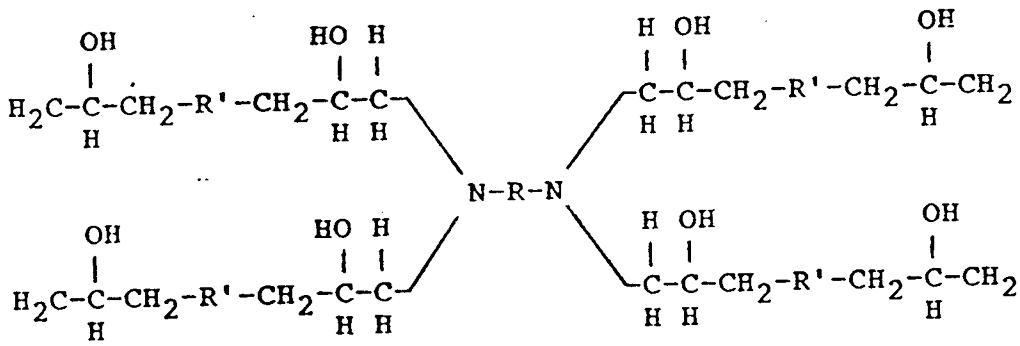

(VIII)

wherein R and R' have the meanings given in Claim 1 or 2.

**4.** A method of making a compound according to Claim 1 or 2 which comprises:
a) mixing a solution of a diglycidyl ether having the formula:

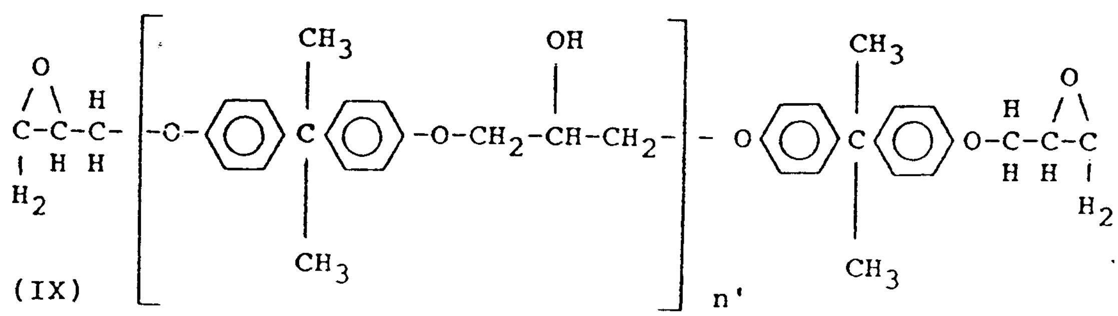

(IX)

wherein n' has the meaning given in Claim 1, in acetone or methyl ethyl ketone as solvent with a polyoxyalkylene diamine of the formula

$H_2N\text{-}R\text{-}NH_2$

wherein R has the meaning given in Claim 1, in a molar ratio of from 4 to 5 moles of diglycidyl ether per mole of polyoxyalkylene diamine, and
b) heating the resulting mixture with agitation at a temperature of 80 to 160°C for 1 to 4 hours, to react said diglycidyl ether quantitatively with said polyoxyalkylene diamine, and to volatilize the solvent.

**5.** A method according to Claim 4 characterized in that the diamine has the formula:

(X)

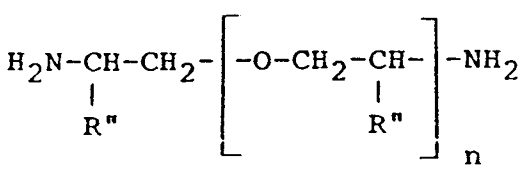

wherein n and R" have the meanings given in Claim 1.

**6.** A method according to Claim 4 characterized in that the diamine is a polyoxyalkylene diamine having the formula:

(XI) 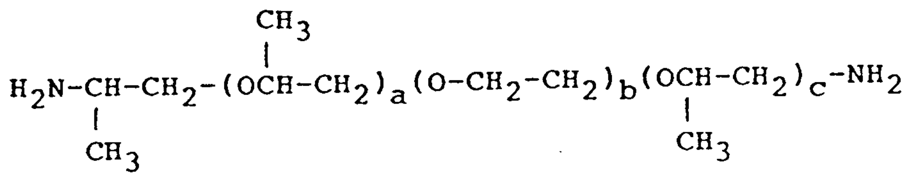

wherein a, b and c have the meanings given in Claim 1.

**7.** A method according to Claim 4 characterized in that the diamine is a fatty acid dimer diamine containing about 36 carbon atoms.

**8.** A method according to any one of Claims 4 to 7 characterized in that, after reaction of the glycidyl ether and the diamine, and volatilization of the solvent, a polyoxyethylene diamine having the formula:

(XII) $H_2N\text{-}CH_2\text{-}CH_2\text{-}[\text{-}O\text{-}CH_2\text{-}CH_2\text{-}]_d\text{-}NH_2$

wherein d has the meaning given in Claim 3, is added in a mole ratio from 4 to 10 mols of polyoxyethylene diamine per mole of diamine initially charged, followed by heating at a temperature of 100 to 150 °C for 0.5 to 5 hours, to form an amine terminated derivative according to Claim 3.

European Patent Office

# EUROPEAN SEARCH REPORT

Application Number

EP 91 30 3632

## DOCUMENTS CONSIDERED TO BE RELEVANT

| Category | Citation of document with indication, where appropriate, of relevant passages | Relevant to claim | CLASSIFICATION OF THE APPLICATION (Int. Cl.5) |
|---|---|---|---|
| X | US-A-4 940 770 (SPERANZA et al.)<br>* Column 4, lines 4-47; column 5, line 55 - column 7, line 25; column 7, lines 35-36 and 53-56; column 10, lines 3-11; examples 17,18,20,24,25; claim 6 *<br>--- | 1-6,8 | C 08 G 59/10<br>C 08 G 59/18<br>C 08 G 59/32<br>C 08 L 63/00 |
| A | GB-A-1 185 375 (MINNESOTA MINING AND MANUFACTURING CO.)<br>* Page 1, line 43 - page 5, line 35; claims 1-4,6,7-9,21-24 *<br>--- | 1 | |
| E | US-A-5 025 100 (LIN et al.)<br>* The whole document *<br>--- | 1-6,8 | |
| A | US-I- 489 331 (SCHREIBER et al.)<br>* The whole document *<br>----- | 1 | |
| | | | TECHNICAL FIELDS SEARCHED (Int. Cl.5) |
| | | | C 08 G<br>C 08 L |

The present search report has been drawn up for all claims

| Place of search | Date of completion of the search | Examiner |
|---|---|---|
| THE HAGUE | 06-04-1992 | NIAOUNAKIS M. |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

European Patent Office

## CLAIMS INCURRING FEES

The present European patent application comprised at the time of filing more than ten claims.

- [ ] All claims fees have been paid within the prescribed time limit. The present European search report has been drawn up for all claims.
- [ ] Only part of the claims fees have been paid within the prescribed time limit. The present European search report has been drawn up for the first ten claims and for those claims for which claims fees have been paid,

  namely claims:
- [ ] No claims fees have been paid within the prescribed time limit. The present European search report has been drawn up for the first ten claims.

## LACK OF UNITY OF INVENTION

The Search Division considers that the present European patent application does not comply with the requirement of unity of invention and relates to several inventions or groups of inventions,
namely:

see sheet -B-

- [x] All further search fees have been paid within the fixed time limit. The present European search report has been drawn up for all claims.
- [ ] Only part of the further search fees have been paid within the fixed time limit. The present European search report has been drawn up for those parts of the European patent application which relate to the inventions in respect of which search fees have been paid,

  namely claims:
- [ ] None of the further search fees has been paid within the fixed time limit. The present European search report has been drawn up for those parts of the European patent application which relate to the invention first mentioned in the claims,

  namely claims:

EP 91 30 3632 -B-

European Patent Office

## LACK OF UNITY OF INVENTION

The Search Division considers that the present European patent application does not comply with the requirement of unity of invention and relates to several inventions or groups of inventions,
namely:

1. Claims 1-6,8: Tetraglycidylethers of formula I of claim 1, where R is the residue of a polyoxyalkylene diamine.

2. Claims 1,3,7,8: Tetraglycidylethers of formula I of claim 1, where R is the residue of a fatty acid dimer diamine.